# EUROPEAN PATENT SPECIFICATION

(11) **EP 3 541 775 B1**
(45) Date of publication and mention of the grant of the patent: **27.07.2022**
(21) Application number: 17797661.0
(22) Date of filing: 16.11.2017
(51) Int. Cl.: C07C 45/51, C07C 49/20, C07C 49/203

(54) **NOVEL PROCESS FOR THE MANUFACTURE OF GAMMA,DELTA-UNSATURATED KETONES**
NEUARTIGES VERFAHREN ZUR HERSTELLUNG VON GAMMA,DELTA-UNGESÄTTIGTEN KETONEN
NOUVEAU PROCÉDÉ DE FABRICATION DE CÉTONES GAMMA, DELTA-INSATURÉES

(30) Priority: 18.11.2016 EP 16199438
(43) Date of publication of application: 25.09.2019
(73) Proprietor: DSM IP Assets B.V., 6411 TE Heerlen (NL)
(72) Inventor: AQUINO, Fabrice, 4303 Kaiseraugst (CH); BONRATH, Werner, 4303 Kaiseraugst (CH); RIEBEL, Peter Hans, 4303 Kaiseraugst (CH)
(74) Representative: Dux, Roland
(86) International application number: PCT/EP2017/079524
(87) International publication number: WO 2018/091622

(56) References cited:
- WO-A1-98/23570
- WO-A2-2010/046199

## Description

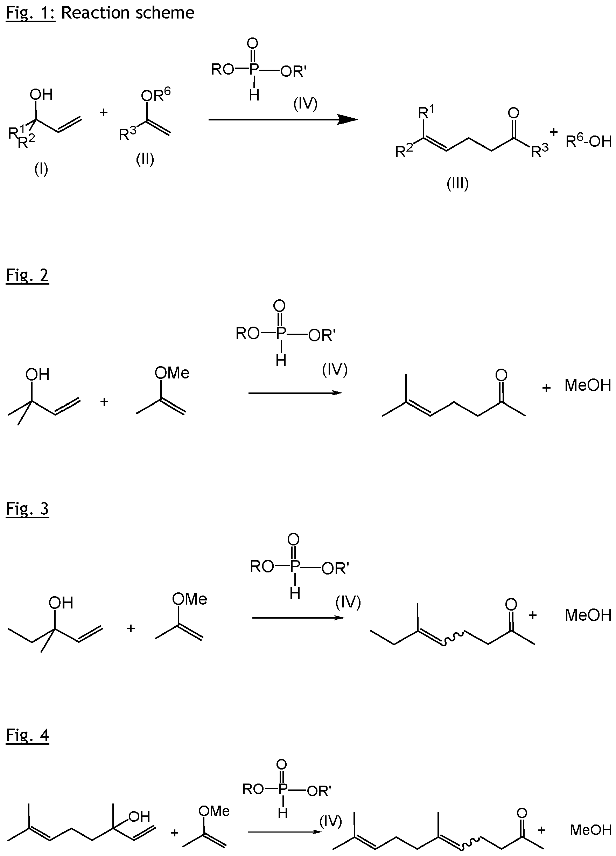

The present invention is directed towards an industrial sustainable process for the manufacture of gamma,delta-unsaturated ketones of formula (III) in the presence of novel catalysts of formula (IV), 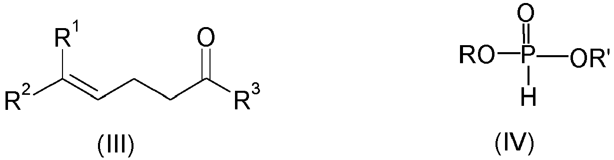 wherein R¹ is methyl or ethyl, R³ is methyl, R² is a saturated or unsaturated linear, branched or cyclic hydrocarbyl group with 1 to 46 C atoms, and R and R' are independently from each other selected from the group consisting of linear C₁₋₆-alkyl, branched C₃₋₆-alkyl, aryl and aralkyl (see also **Fig. 1****).**

WO 98/23570 A1 discloses a process of preparing compounds of the formula (II) using phosphonic acids or phosphinic acids or phosphonates as catalysts. WO 2010/046199 A2 discloses a process of preparing compounds of the formula (II) using ammonium salt as catalysts.

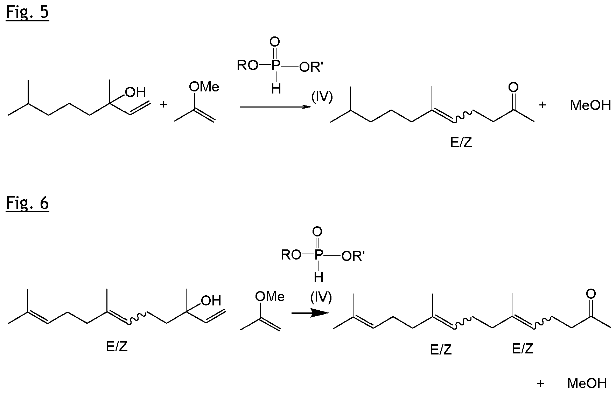

The present invention is especially directed towards an industrial sustainable process for the manufacture of 6-methyl-5-hepten-2-one ("MH"; see **Fig. 2****),** 6-methyl-5-octen-2-one ("EH"; see **Fig. 3****),** (5EZ)-6,10-dimethyl-5,9-undecadien-2-one (E)-geranylacetone = "GA"; (Z)-nerylacetone = "NA"; see **Fig. 4****),** (5*EZ*)-6,10-dimethyl-5-undecen-2-one ((E)-dihydrogeranyl-acetone = "DHGA"; (Z)-dihydronerylacetone = "DHNA"; see **Fig. 5)** and 6,10,14-trimethyl-5,9,13-pentadeca-trien-2-one (= farnesylacetone; "FA"; see **Fig. 6**).

Preferred catalysts according to the present invention are "DPPI" (diphenyl phosphite), "DMPI" (dimethyl phosphite) and "DBPI" (dibenzyl phosphite).

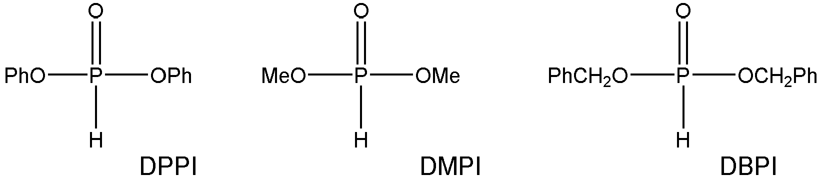

### Detailed description

The present invention is directed towards a process for the manufacture of gamma,delta-unsaturated ketones of the general formula (III) 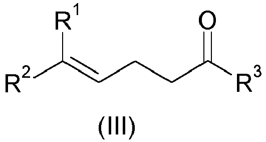 by reacting a tertiary vinyl carbinol of the general formula (I) with an isopropenyl alkyl ether of the general formula (II) in the presence of a catalyst of the general formula (IV), 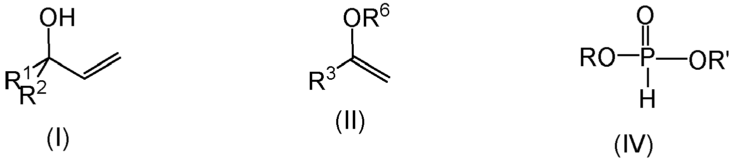 wherein R¹ and R⁶ are independently from each other methyl or ethyl, R³ is methyl, R² is a saturated or unsaturated linear, branched or cyclic hydrocarbyl group with 1 to 46 C atoms, and
R is selected from the group consisting of linear C₁₋₆-alkyl, branched C₃₋₆-alkyl, aryl and aralkyl.

### Starting materials

Compound of the formula (I) 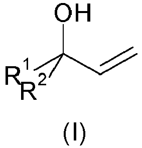 R¹ is either methyl or ethyl, and R² is a saturated or unsaturated linear, branched or cyclic hydrocarbyl group with 1 to 46 C atoms.

Preferred examples of R² are listed in the following **Table 1**.

| R² | |
|---|---|
| methyl | CH₃ |
| ethyl | CH₂CH₃ |
| 4-methylpentyl | 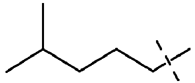 |
| CH₂-prenyl = "homoprenyl" | 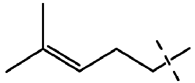 |
| CH₂-geranyl/CH₂-neryl = "homogeranyl"/" homoneryl" | 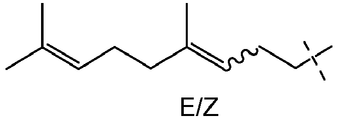 |
| CH₂-farnesyl = "homofarnesyl" | 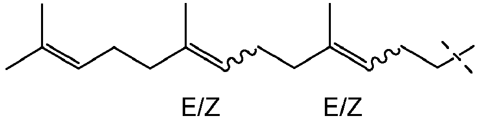 |
| CH₂-hexahydrofarnesyl | 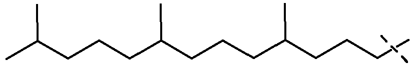 |
| CH₂-solanesyl = "homosolanesyl" | 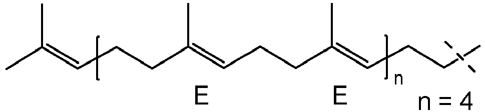 |
| 2-(2',6',6'-trimethylcyclohex-1'-en-1'-yl)ethen-1-yl | 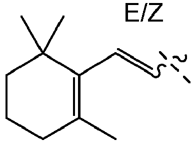 |

The compound of the general formula (I) is preferably selected from the group consisting of 2-methyl-3-buten-2-ol ("MBE"), 3-methyl-1-penten-3-ol ("EBE"), 3,7-dimethyl-1,6-octadien-3-ol (= linalool, "LL"), 3,7-dimethyl-1-octen-3-ol ("DMOE"), and (6E/6Z)-3,7,11-trimethyl-1,6,10-dodecatrien-3-ol (= E/Z-nerolidol, "E/Z-NL").

More preferably the compound of formula (I) is MBE or EBE.

### Compound of the formula (II)

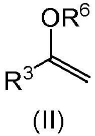

The compound of formula (II) is preferably either isopropenyl methyl ether ("IPM") or isopropenyl ethyl ether ("IPE"), whereby isopropenyl methyl ether is more preferred.

### Amounts of compounds of the formula (I) and (II)

The molar ratio of the tertiary vinyl carbinol of the general formula (I) to the isopropenyl alkyl ether of the general formula (II) is preferably ranging from 1:7 to 1:1, more preferably ranging from 1:5 to 1:1.5, most preferably ranging from 1:3.5 to 1:2.

### Catalysts of the formula (IV)

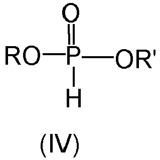

R and R' are independently from each other selected from the group consisting of linear C₁₋₆-alkyl, branched C₃₋₆-alkyl, aryl and aralkyl. Preferably R and R' are independently from each other selected from the group consisting of methyl, ethyl, n-propyl, n-butyl, n-pentyl, n-hexyl, 2-propyl, 2-butyl, 2-methylprop-1-yl, tert-butyl, 2-pentyl, 3-pentyl, neopentyl, 2-methylpent-1-yl, phenyl, 1-naphthyl, 2-naphthyl and benzyl.

More preferred are the catalysts as cited above wherein R and R' are identical.

Even more preferred are catalysts of the formula (IV), wherein R and R' are identical and selected from the group consisting of methyl, ethyl, propyl, n-butyl, phenyl and benzyl. Especially preferred are catalysts of the formula (IV), wherein R and R' are identical and either methyl, n-butyl, phenyl or benzyl, i.e. dimethyl phosphite ("DMPI"), di-n-butyl phosphite ("DNBP"), diphenyl phosphite ("DPPI") and dibenzyl phosphite ("DBPI").

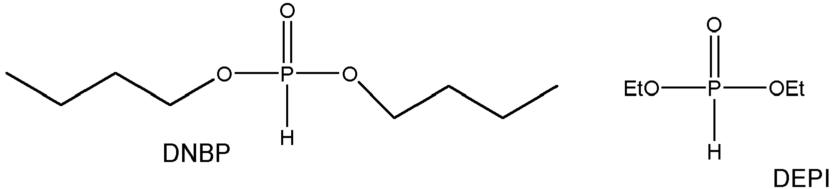

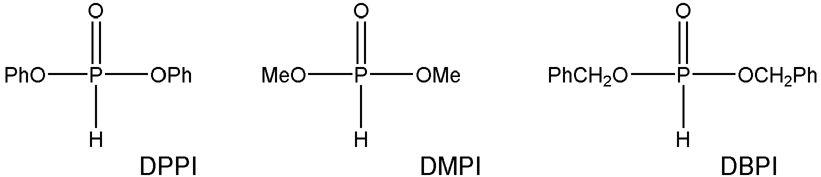

Most preferred are DPPI, DMPI and DBPI.

The amount of the catalyst of formula (IV) is preferably ranging from 0.01 - 0.30 mol-%, more preferably ranging from 0.05-0.25 mol-%, most preferably ranging from 0.07-0.20 mol-%, based on the amount of the tertiary vinyl carbinol of the general formula (I).

Advantageously the catalysts of formula (IV) are used as such, but they may also be used as solution in a suitable organic solvent such as e.g. acetone.

### Reaction conditions

### Temperature

The reaction is preferably carried out at a temperature ranging from 100 to 170°C, more preferably at a temperature ranging from 110 to 160°C, most preferably at a temperature ranging from 120 to 150°C.

### Pressure

The reaction is preferably carried out at a pressure ranging from 5 to 15 bar, more preferably at a pressure ranging from 8 to 12 bar.

### Solvent

The reaction can be carried out without solvent or in the presence of an organic solvent. Preferably the reaction is carried out without solvent.

If the reaction is carried out in an organic solvent, polar aprotic organic solvents such as aliphatic ketones as e.g. acetone are preferred.

Even if the reaction is carried out in the absence of an organic solvent, the starting materials, the compounds of formula (I) and (II), as well as the catalyst of formula (IV) may still be provided in an organic solvent. Thus, there may be an amount of organic solvent up to 10 weight-%, preferably an amount of organic solvent up to 5 weight-%, more preferably an amount of organic solvent up to 3 weight-%, based on the total weight of the reaction mixture, present in the reaction mixture.

The reaction mixture itself, with or without an organic solvent, is also an object of the present invention. Thus, the present invention is directed towards a reaction mixture comprising a compound of formula (I), a compound of formula (II) and a catalyst of formula (IV), as well as towards a reaction mixture consisting essentially of a compound of formula (I), a compound of formula (II) and a catalyst of formula (IV). 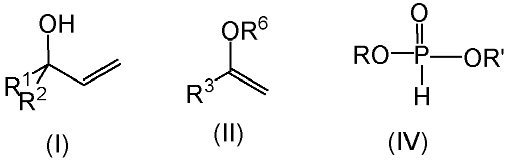 "consisting essentially of" in this context means that the total amount of the compound of formula (I), the compound of formula (II) and the catalyst of formula (IV) sum up to an amount of at least 90 weight-%, preferably at least 95 weight-%, more preferably at least 97 weight-%, based on the total amount of the reaction mixture.

The present invention is also directed towards the use of a catalyst of formula (IV) 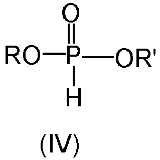 wherein R and R' are independently from each other selected from the group consisting of linear C₁₋₆-alkyl, branched C₃₋₆-alkyl, aryl and aralkyl, preferably R and R' are independently from each other selected from the group consisting of methyl, ethyl, n-propyl, n-butyl, n-pentyl, n-hexyl, 2-propyl, 2-butyl, 2-methylprop-1-yl, tert-butyl, 2-pentyl, 3-pentyl, neopentyl, 2-methylpent-1-yl, phenyl, 1-naphthyl, 2-naphthyl and benzyl, more preferably R and R' are identical and selected from the group consisting of methyl, ethyl, propyl, n-butyl, phenyl and benzyl, even more preferably R and R' are identical and selected from the group consisting of methyl, n-butyl, phenyl or benzyl, most preferably R and R' are identical and either methyl, phenyl or benzyl,
for the manufacture of gamma,delta-unsaturated ketones of the general formula (III) 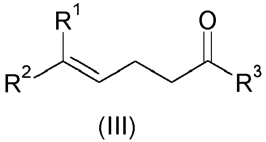
by reacting a tertiary vinyl carbinol of the general formula (I) with an isopropenyl alkyl ether of the general formula (II) 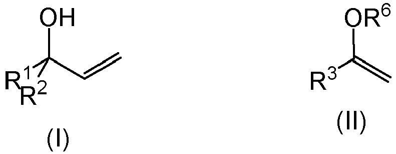
wherein R¹ and R⁶ are independently from each other methyl or ethyl, R³ is methyl, and R² is a saturated or unsaturated linear, branched or cyclic hydrocarbyl group with 1 to 46 C atoms, whereby the preferences as given above also apply.

The present invention is now further illustrated by the following nonlimiting examples.

### Examples:

### General procedure

The powdery catalyst (either DPPI, DMPI or DBPI in the amount as given in table 2 below) is filled into the reactor as such. 144 g of the starting material (MBE) and the amount of IPM ("eq." refers to the molar ratio relative to the other starting material, the compound of formula (I)) as given in table 2 below are mixed and poured into the reactor. The closed reactor is then evacuated and subsequently released with nitrogen three times. The reaction mixture is stirred and heated up to the temperature as given in table 2 below. The experiment is then carried out at isothermal conditions. After the reaction time given in table 2, the reaction mixture is cooled down to 20°C. A sample of the reaction mixture is collected and directly neutralized with sodium acetate. The sample is then analyzed by gas chromatography.

**Table 2:**

| **Example** | **1** | **2** | **3** | **4** | **5** | **6** | **7** | **8** | **9** |
|---|---|---|---|---|---|---|---|---|---|
| Amount of IPM [eq.] | 2.1 | 2.1 | 2.1 | 2.1 | 2.1 | 2.1 | 2.1 | 2.1 | 2.1 |
| Catalyst | DPPI | DPPI | DBPI | DBPI | DPPI | DPPI | DPPI | DMPI | DMPI |
| Amount of catalyst [mol-%], based on MBE | 0.1 | 0.165 | 0.165 | 0.125 | 0.2 | 0.125 | 0.125 | 0.165 | 0.125 |
| Temperature [°C] | 150 | 150 | 150 | 150 | 150 | 150 | 150 | 150 | 150 |
| Reaction time [hours] | 16 | 14.6 | 14.6 | 16 | 9 | 10 | 13 | 14.6 | 16 |
| Conversion MBE [%] | 96.3 | 98.4 | 98.3 | 97.5 | 98.2 | 91.4 | 96.5 | 98.8 | 98.0 |
| Yield MH [%] | 94.5 | 92.3 | 94.2 | 93.0 | 92.3 | 89.0 | 93.5 | 95.1 | 93.9 |
| Selectivity | 0.98 | 0.94 | 0.96 | 0.95 | 0.94 | 0.97 | 0.97 | 0.96 | 0.96 |

## Claims

1. A process for the manufacture of gamma,delta-unsaturated ketones of the general formula (III) 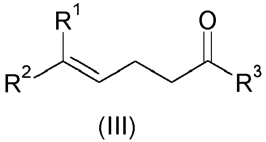 by reacting a tertiary vinyl carbinol of the general formula (I) with an isopropenyl alkyl ether of the general formula (II) in the presence of a catalyst of the general formula (IV), 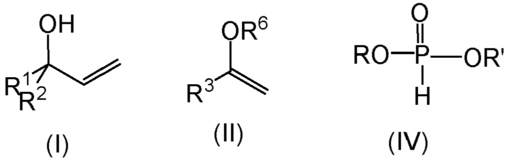 wherein R¹ and R⁶ are independently from each other methyl or ethyl, R³ is methyl,
R² is a saturated or unsaturated linear, branched or cyclic hydrocarbyl group with 1 to 46 C atoms, and
R and R' are independently selected from each other from the group consisting of linear C₁₋₆-alkyl, branched C₃₋₆-alkyl, aryl and aralkyl.

2. The process according to claim 1, wherein R and R' are identical and selected from the group consisting of methyl, ethyl, propyl, n-butyl, phenyl and benzyl, preferably wherein R and R' are identical and either methyl, phenyl or benzyl.

3. The process according to any one or more of the preceding claims, wherein the reaction is carried out at a temperature ranging from 100 to 170°C, preferably at a temperature ranging from 110 to 160°C, more preferably at a temperature ranging from 120 to 150°C.

4. The process according to any one or more of the preceding claims, wherein the reaction is carried out at a pressure ranging from 5 to 15 bar, preferably at a pressure ranging from 8 to 12 bar.

5. The process according to any one or more of the preceding claims, wherein the molar ratio of the tertiary vinyl carbinol of the general formula (I) to the isopropenyl alkyl ether of the general formula (II) is ranging from 1:7 to 1:1, preferably ranging from 1:5 to 1:2, more preferably ranging from 1:3.5 to 1:2.

6. The process according to any one or more of the preceding claims, wherein the amount of the catalyst is ranging from 0.01-0.3 mol-%, preferably ranging from 0.02-0.1 mol-%, more preferably ranging from 0.02-0.07 mol-%, based on the amount of the tertiary vinyl carbinol of the general formula (I).

7. The process according to any one or more of the preceding claims, wherein the compound of the general formula (III) is selected from the group consisting of 6-methyl-5-hepten-2-one, 6-methyl-5-octen-2-one, 6,10-dimethyl-5,9-undecadien-2-one, 6,10-dimethyl-5-undecen-2-one and 6,10,14-trimethyl-5,9,13-pentadeca-trien-2-one, preferably wherein the compound of the general formula (III) is 6-methyl-5-hepten-2-one.

8. Use of a catalyst of the formula (IV) 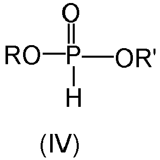 wherein R and R' are independently selected from each other from the group consisting of linear C₁₋₆-alkyl, branched C₃₋₆-alkyl, aryl and aralkyl, preferably wherein R and R' are identical and selected from the group consisting of methyl, ethyl, propyl, n-butyl, phenyl and benzyl, preferably wherein R and R' are identical and either methyl, phenyl or benzyl,
for the manufacture of gamma,delta-unsaturated ketones of the general formula (III) 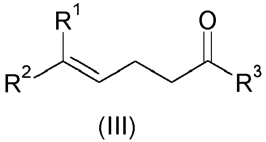 by reacting a tertiary vinyl carbinol of the general formula (I) with an isopropenyl alkyl ether of the general formula (II) 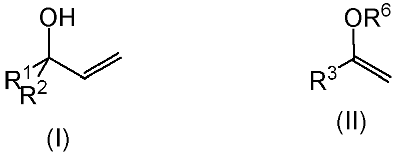 wherein R¹ and R⁶ are independently from each other methyl or ethyl, R³ is methyl, and R² is a saturated or unsaturated linear, branched or cyclic hydrocarbyl group with 1 to 46 C atoms.

9. A reaction mixture comprising a compound of formula (I), a compound of formula (II) and a catalyst of formula (IV) 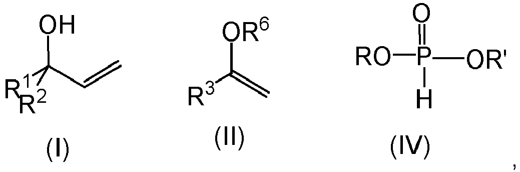 wherein R¹ and R⁶ are independently from each other methyl or ethyl, R³ is methyl, R² is a saturated or unsaturated linear, branched or cyclic hydrocarbyl group with 1 to 46 C atoms, and
R and R' are independently from each other selected from the group consisting of linear C₁₋₆-alkyl, branched C₃₋₆-alkyl, aryl and aralkyl.

10. A reaction mixture according to claim 9 consisting essentially of a compound of formula (I), a compound of formula (II) and a catalyst of formula (IV) 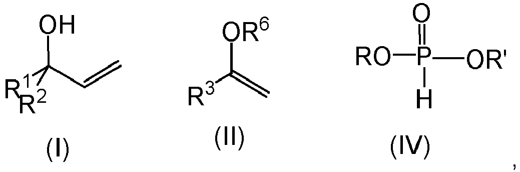 wherein R¹ and R⁶ are independently from each other methyl or ethyl, R³ is methyl, R² is a saturated or unsaturated linear, branched or cyclic hydrocarbyl group with 1 to 46 C atoms, and
R and R' are independently from each other selected from the group consisting of linear C₁₋₆-alkyl, branched C₃₋₆-alkyl, aryl and aralkyl.

## Patentansprüche

1. Verfahren zur Herstellung von gamma,delta-ungesättigten Ketonen der allgemeinen Formel (III) 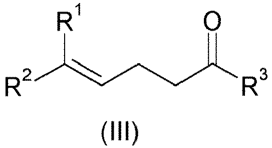 durch Umsetzen eines tertiären Vinylcarbinols der allgemeinen Formel (I) mit einem Isopropenylalkylether der allgemeinen Formel (II) in Gegenwart eines Katalysators der allgemeinen Formel (IV), 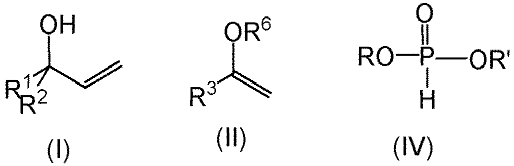 wobei R¹ und R⁶ unabhängig voneinander für Methyl oder Ethyl stehen,
R³ für Methyl steht,
R² für eine gesättigte oder ungesättigte lineare, verzweigte oder cyclische Hydrocarbylgruppe mit 1 bis 46 C-Atomen steht und
R und R' unabhängig voneinander aus der Gruppe bestehend aus linearem C₁₋₆-Alkyl, verzweigtem C₃₋₆-Alkyl, Aryl und Aralkyl ausgewählt sind.

2. Verfahren nach Anspruch 1, wobei R und R' gleich sind und aus der Gruppe bestehend aus Methyl, Ethyl, Propyl, n-Butyl, Phenyl und Benzyl ausgewählt sind, vorzugsweise wobei R und R' gleich sind und für Methyl, Phenyl oder Benzyl stehen.

3. Verfahren nach einem oder mehreren der vorhergehenden Ansprüche, wobei die Umsetzung bei einer Temperatur im Bereich von 100 bis 170 °C, vorzugsweise bei einer Temperatur im Bereich von 110 bis 160 °C, weiter bevorzugt bei einer Temperatur im Bereich von 120 bis 150 °C, durchgeführt wird.

4. Verfahren nach einem oder mehreren der vorhergehenden Ansprüche, wobei die Umsetzung bei einem Druck im Bereich von 5 bis 15 bar, vorzugsweise bei einem Druck im Bereich von 8 bis 12 bar, durchgeführt wird.

5. Verfahren nach einem oder mehreren der vorhergehenden Ansprüche, wobei das Molverhältnis von tertiärem Vinylcarbinol der allgemeinen Formel (I) zu Isopropenylalkylether der allgemeinen Formel (II) im Bereich von 1:7 bis 1:1, vorzugsweise im Bereich von 1:5 bis 1:2, weiter bevorzugt im Bereich von 1:3,5 bis 1:2, liegt.

6. Verfahren nach einem oder mehreren der vorhergehenden Ansprüche, wobei die Menge des Katalysators im Bereich von 0,01-0,3 Mol-%, vorzugsweise im Bereich von 0,02-0,1 Mol-%, weiter bevorzugt im Bereich von 0,02-0,07 Mol-%, bezogen auf die Menge des tertiären Vinylcarbinols der allgemeinen Formel (I), liegt.

7. Verfahren nach einem oder mehreren der vorhergehenden Ansprüche, wobei die Verbindung der allgemeinen Formel (III) aus der Gruppe bestehend aus 6-Methyl-5-hepten-2-on, 6-Methyl-5-octen-2-on, 6,10-Dimethyl-5,9-undecadien-2-on, 6,10-Dimethyl-5-undecen-2-on und 6,10,14-Trimethyl-5,9,13-pentadecatrien-2-on ausgewählt wird, vorzugsweise wobei es sich bei der Verbindung der allgemeinen Formel (III) um 6-Methyl-5-hepten-2-on handelt.

8. Verwendung eines Katalysators der Formel (IV) 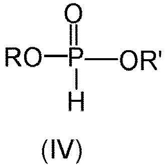 wobei R und R' unabhängig voneinander aus der Gruppe bestehend aus linearem C₁₋₆-Alkyl, verzweigtem C₃₋₆-Alkyl, Aryl und Aralkyl ausgewählt sind, vorzugsweise wobei R und R' gleich sind und aus der Gruppe bestehend aus Methyl, Ethyl, Propyl, n-Butyl, Phenyl und Benzyl ausgewählt sind, vorzugsweise wobei R und R' gleich sind und für Methyl, Phenyl oder Benzyl stehen,
zur Herstellung von gamma,delta-ungesättigten Ketonen der allgemeinen Formel (III) 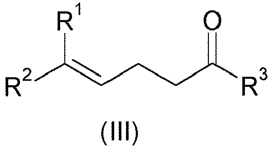 durch Umsetzen eines tertiären Vinylcarbinols der allgemeinen Formel (I) mit einem Isopropenylalkylether der allgemeinen Formel (II) 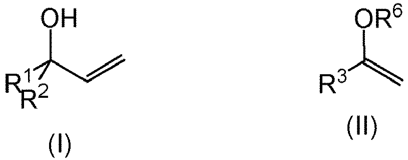 wobei R¹ und R⁶ unabhängig voneinander für Methyl oder Ethyl stehen, R³ für Methyl steht und R² für eine gesättigte oder ungesättigte lineare, verzweigte oder cyclische Hydrocarbylgruppe mit 1 bis 46 C-Atomen steht.

9. Reaktionsmischung, umfassend eine Verbindung der Formel (I), eine Verbindung der Formel (II) und einen Katalysator der Formel (IV) 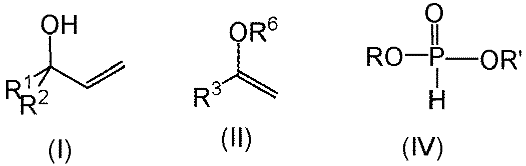 wobei R¹ und R⁶ unabhängig voneinander für Methyl oder Ethyl stehen, R³ für Methyl steht, R² für eine gesättigte oder ungesättigte lineare, verzweigte oder cyclische Hydrocarbylgruppe mit 1 bis 46 C-Atomen steht und
R und R' unabhängig voneinander aus der Gruppe bestehend aus linearem C₁₋₆-Alkyl, verzweigtem C₃₋₆-Alkyl, Aryl und Aralkyl ausgewählt sind.

10. Reaktionsmischung nach Anspruch 9, im Wesentlichen bestehend aus einer Verbindung der Formel (I), einer Verbindung der Formel (II) und einem Katalysator der Formel (IV) 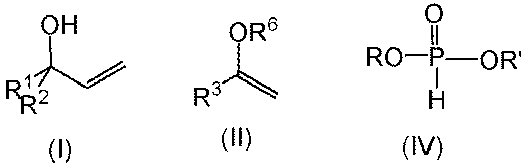 wobei R¹ und R⁶ unabhängig voneinander für Methyl oder Ethyl stehen, R³ für Methyl steht, R² für eine gesättigte oder ungesättigte lineare, verzweigte oder cyclische Hydrocarbylgruppe mit 1 bis 46 C-Atomen steht und
R und R' unabhängig voneinander aus der Gruppe bestehend aus linearem C₁₋₆-Alkyl, verzweigtem C₃₋₆-Alkyl, Aryl und Aralkyl ausgewählt sind.

## Revendications

1. Procédé pour la fabrication de cétones gamma,delta-insaturées de la formule générale (III) 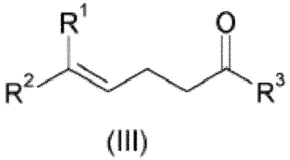 par mise en réaction d'un vinylcarbinol tertiaire de la formule générale (I) avec un éther d'alkyle et d'isopropényle de la formule générale (II) en la présence d'un catalyseur de la formule générale (IV), 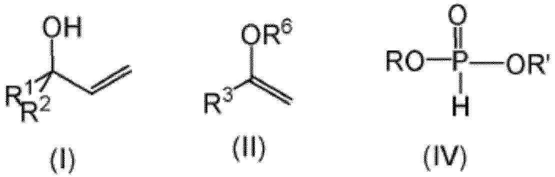 R¹ et R⁶ étant indépendamment l'un de l'autre méthyle ou éthyle,
R³ étant méthyle,
R² étant un groupe hydrocarbyle saturé ou insaturé, linéaire, ramifié ou cyclique comportant 1 à 46 atomes de C, et
R et R' étant choisis indépendamment l'un de l'autre dans le groupe constitué par C₁₋₆-alkyle linéaire, C₃₋₆-alkyle ramifié, aryle et aralkyle.

2. Procédé selon la revendication 1, R et R' étant identiques et choisis dans le groupe constitué par méthyle, éthyle, propyle, n-butyle, phényle et benzyle, préférablement R et R' étant identiques et soit méthyle, soit phényle, soit benzyle.

3. Procédé selon l'une quelconque des revendications précédentes, la réaction étant mise en œuvre à une température dans la plage de 100 à 170 °C, préférablement à une température dans la plage de 110 à 160 °C, plus préférablement à une température dans la plage de 120 à 150 °C.

4. Procédé selon l'une quelconque des revendications précédentes, la réaction étant mise en œuvre à une pression dans la plage de 5 à 15 bars, préférablement à une pression dans la plage de 8 à 12 bars.

5. Procédé selon l'une quelconque des revendications précédentes, le rapport molaire du vinylcarbinol tertiaire de la formule générale (I) sur l'éther d'alkyle et d'isopropényle de la formule générale (II) étant dans la plage de 1 : 7 à 1 : 1, préférablement dans la plage de 1 : 5 à 1 : 2, plus préférablement dans la plage de 1 : 3,5 à 1 : 2.

6. Procédé selon l'une quelconque des revendications précédentes, la quantité du catalyseur étant dans la plage de 0,01 à 0,3 % en moles, préférablement dans la plage de 0,02 à 0,1 % en moles, plus préférablement dans la plage de 0,02 à 0,07 % en moles, sur la base de la quantité du vinylcarbinol tertiaire de la formule générale (I).

7. Procédé selon l'une quelconque des revendications précédentes, le composé de la formule générale (III) étant choisi dans le groupe constitué par la 6-méthyl-5-heptén-2-one, la 6-méthyl-5-octén-2-one, la 6,10-diméthyl-5,9-undécadién-2-one, la 6,10-diméthyl-5-undécén-2-one et la 6,10,14-triméthyl-5,9,13-pentadéca-trién-2-one, préférablement le composé de la formule générale (III) étant la 6-méthyl-5-heptén-2-one.

8. Utilisation d'un catalyseur de la formule (IV) 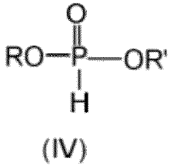 R et R' étant choisis indépendamment l'un de l'autre dans le groupe constitué par C₁₋₆-alkyle linéaire, C₃₋₆-alkyle ramifié, aryle et aralkyle, préférablement R et R' étant identiques et choisis dans le groupe constitué par méthyle, éthyle, propyle, n-butyle, phényle et benzyle, préférablement R et R' étant identiques et soit méthyle, soit phényle, soit benzyle,
pour la fabrication de de cétones gamma,delta-insaturées de la formule générale (III) 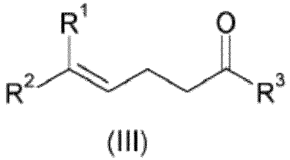 par mise en réaction d'un vinylcarbinol tertiaire de la formule générale (I) avec un éther d'alkyle et d'isopropényle de la formule générale (II) 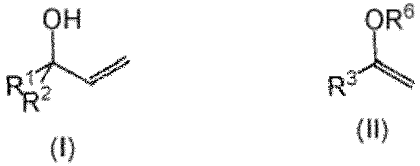 R¹ et R⁶ étant indépendamment l'un de l'autre méthyle ou éthyle, R³ étant méthyle, R² étant un groupe hydrocarbyle saturé ou insaturé, linéaire, ramifié ou cyclique comportant 1 à 46 atomes de C.

9. Mélange réactionnel comprenant un composé de formule (I), un composé de formule (II) et un catalyseur de formule (IV) 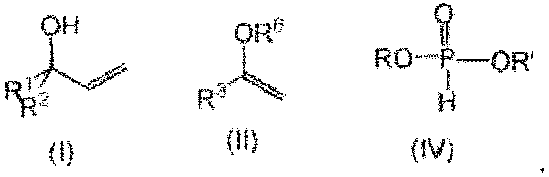 R¹ et R⁶ étant indépendamment l'un de l'autre méthyle ou éthyle, R³ étant méthyle, R² étant un groupe hydrocarbyle saturé ou insaturé, linéaire, ramifié ou cyclique comportant 1 à 46 atomes de C, et
R et R' étant choisis indépendamment l'un de l'autre dans le groupe constitué par C₁₋₆-alkyle linéaire, C₃₋₆-alkyle ramifié, aryle et aralkyle.

10. Mélange réactionnel selon la revendication 9 essentiellement constitué d'un composé de formule (I), d'un composé de formule (II) et d'un catalyseur de formule (IV) 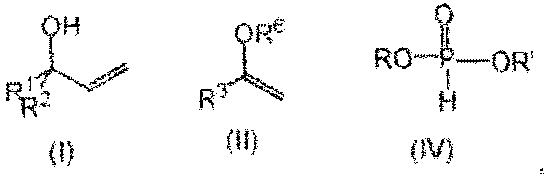 R¹ et R⁶ étant indépendamment l'un de l'autre méthyle ou éthyle, R³ étant méthyle, R² étant un groupe hydrocarbyle saturé ou insaturé, linéaire, ramifié ou cyclique comportant 1 à 46 atomes de C, et
R et R' étant choisis indépendamment l'un de l'autre dans le groupe constitué par C₁₋₆-alkyle linéaire, C₃₋₆-alkyle ramifié, aryle et aralkyle.
